(19) Europäisches Patentamt
European Patent Office
Office européen des brevets

(11) Publication number: 0 004 754 B1

(12) EUROPEAN PATENT SPECIFICATION

(45) Date of publication of patent specification: 05.10.83

(21) Application number: 79300524.0

(22) Date of filing: 30.03.79

(51) Int. Cl.[3]: C 07 C 131/00, C 07 D 317/58, C 07 D 317/54, C 07 D 209/48, C 07 D 307/42, A 01 N 43/38, A 01 N 33/24, A 01 N 43/40, A 01 N 43/06

# (54) Ketoximinoether insecticides.

(30) Priority: 31.03.78 US 891991

(43) Date of publication of application: 17.10.79 Bulletin 79/21

(45) Publication of the grant of the patent: 05.10.83 Bulletin 83/40

(84) Designated Contracting States: BE CH DE FR GB IT NL

(56) References cited:
US - A - 4 079 149

(73) Proprietor: RHONE-POULENC AGROCHIMIE
14-20, rue Pierre Baizet
F-69009 Lyon (FR)

(72) Inventor: Paul, Jill Helaine
1317 River Road
Edgewater Bergen New Jersey 07020 (US)
Inventor: Strong, Jerry Glenn
3 Katherine Drive
Warren Somerset New Jersey 07060 (US)

(74) Representative: Chaumette, Michel et al,
RHONE-POULENC AGROCHIMIE BP 9163-09
F-69263 Lyon Cedex 1 (FR)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 004 754 B1

## Ketoximinoether insecticides

This invention is directed to certain ketoximinoethers having insecticidal activity.

The compounds of this invention have the general structure

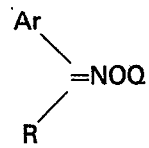

wherein Ar, R and Q are as defined hereinafter.

In U.S. Patent No. 3,980,799, there are disclosed, as synergists for insecticides and acaricides, compounds having the formula:

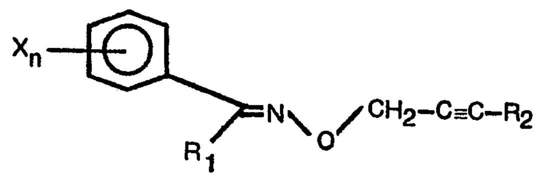

wherein $X_n$ is halogen, alkyl, methylenedioxy, etc., $R_1$ is $C_1$ to $C_4$ alkyl, and $R_2$ is hydrogen or halogen. In British Patent No. 960,241, there are disclosed as synergists for insecticides, compounds having the formula:

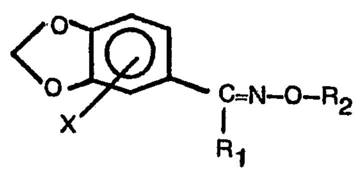

wherein X is hydrogen, n-propyl or alkyl, $R_1$ is hydrogen $C_1$—$C_8$ alkyl or phenyl, and $R_2$ is alkyl, alkenyl, aralkyl or cycloalkyl.

This invention provides compounds having the formula:

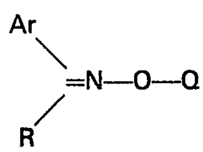

wherein Ar is phenyl; phenyl substituted in the 2- to 6-position with 1—5 halogen (Cl, Br, F, I), $C_1$—$C_4$ alkyl, amino, alkylamino, dialkylamino, alkoxy, alkylthio, methylenedioxy, ethylenedioxy, isopropylenedioxy, trifluoromethyl, haloalkyl, alkylthioalkyl, alkenyl, alkynyl, cyano, cyanoalkyl, carboalkoxy, alkylsulfonyl, alkoxyalkyl, haloalkenyl or acyl; indanyl; naphthyl; benzofuryl; benzodihydrofuryl; benzothienyl; heterocyclic aryl or heterocyclic aryl substituted with halogen, alkyl, nitro, amino, alkylamino, dialkylamino, alkoxy, methylenedioxy, alkylthio, haloalkyl, alkylthioalkyl, alkenyl, alkynyl, cyano, cyanoalkyl, carboalkoxy, alkylsulfonyl, alkoxyalkyl, haloalkenyl or acyl; R is $C_2$—$C_6$ alkyl, $C_3$—$C_6$ cycloalkyl or $C_2$—$C_6$ alkenyl any of which can be substituted by halogen, hydroxy, alkoxy, alkylthio, alkylsulfonyl, cyano, nitro, carboalkoxy or acyl; and Q is:

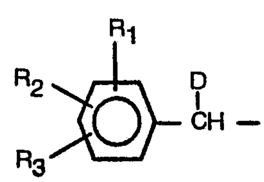

wherein D is hydrogen or alkyl; $R_1$ and $R_2$ are the same or different and are hydrogen, halogen, alkyl, alkenyl, alkynyl, methylenedioxy, haloalkyl, haloalkenyl or cyano; and $R_3$ is —ZY wherein Z is oxygen,

sulfur or methylene and Y is hydrogen, alkyl, alkenyl, alkynyl or phenyl on which can be substituted alkyl, alkenyl, alkoxy, chlorine, bromine or fluorine; or Q is

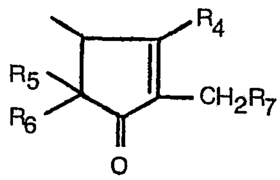

wherein $R_4$ is hydrogen or methyl; $R_5$ and $R_6$ are the same or different and are hydrogen or alkyl; and $R_7$ is ethenyl, ethynyl or butadienyl; or Q is

$$R_8CH_2—$$

wherein $R_8$ is phthalimido, di- or tetra-hydrophthalimido or cyano; or Q is

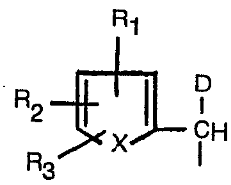

wherein X is oxygen or sulfur; $R_3$ is hydrogen or —ZY and $R_1$, $R_2$, D, Z and Y are defined as above provided that when Q is an $\alpha$-alkyl 3-phenoxybenzyl and R is alkyl or alkenyl of from two to six carbon atoms, optionally substituted by from one to a plurality of halogen atoms or is cyclopropyl, optionally substituted by one or two halogen atoms, Ar cannot be phenyl or phenyl substituted by one or two halogen atoms, alkyl of from one to four carbon atoms or alkoxy of from one to three carbon atoms, and provided also that, when Ar is an optionally substituted phenyl radical, and, at the same time Q is the 3-phenoxybenzyl radical and R is alkyl or alkenyl of from two to six carbon atoms optionally substituted by from one to a plurality of halogen atoms, or is cyclopropyl, optionally substituted by one or two halogen atoms, Ar is a phenyl radical substituted by a trifluoromethyl radical or a phenyl radical substituted by at least one alkoxy group and at least one alkyl group.

Typical examples of the Q substituent are:

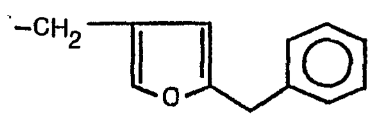
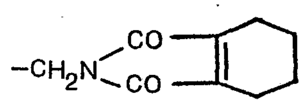
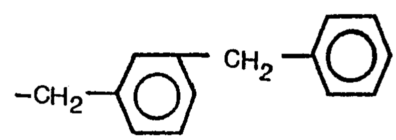

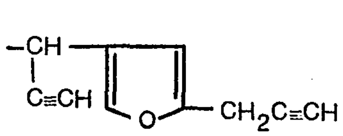
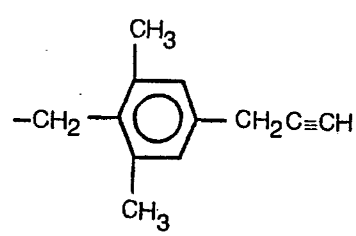
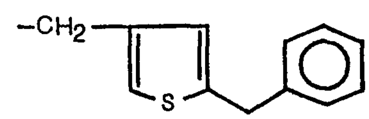

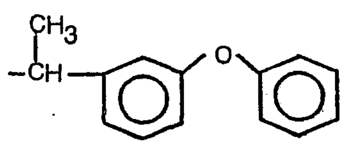
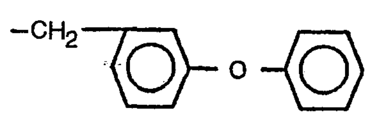
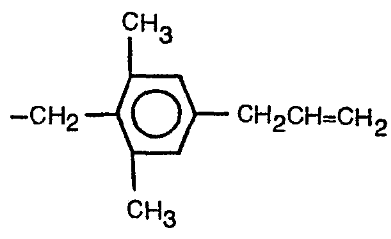

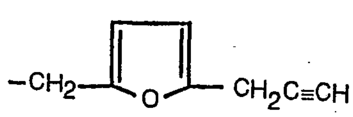
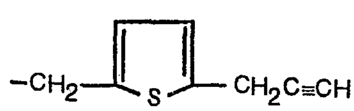
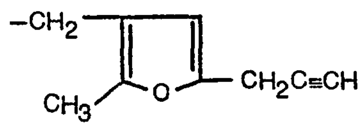

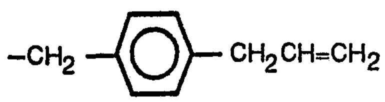
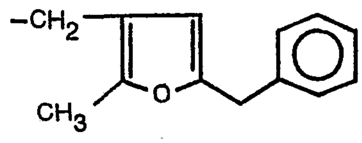
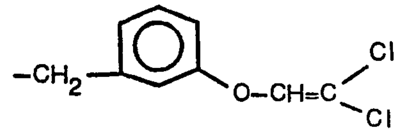

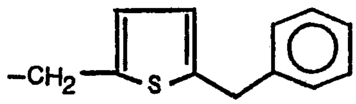
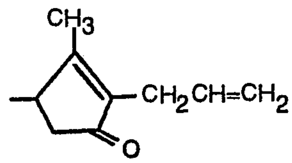
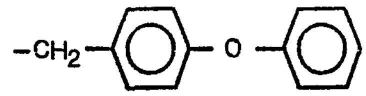

Examples of compounds of this invention are compounds having the structural formulae:

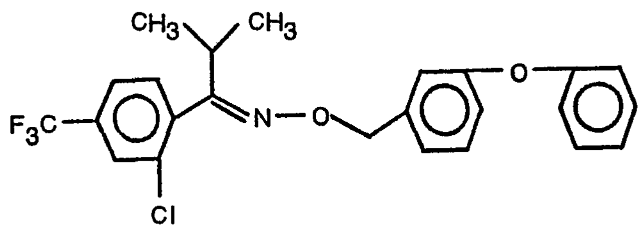

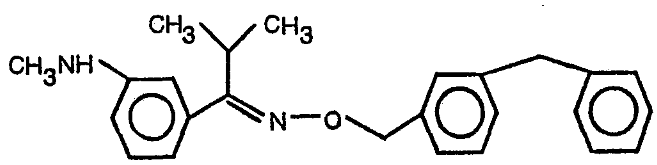

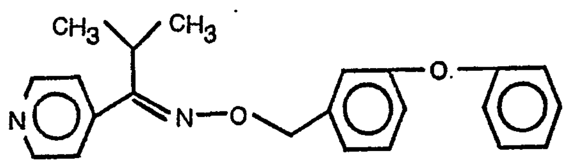

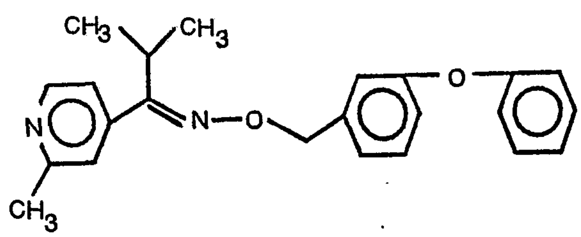

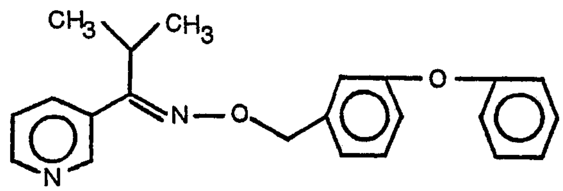

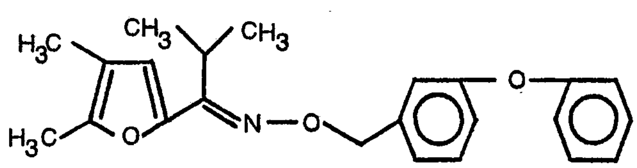

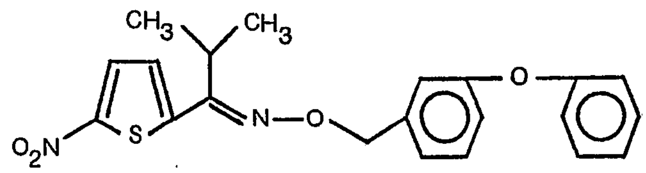

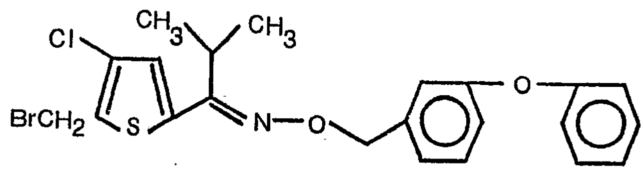

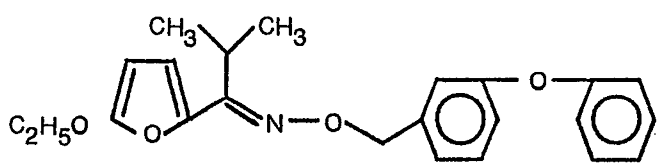

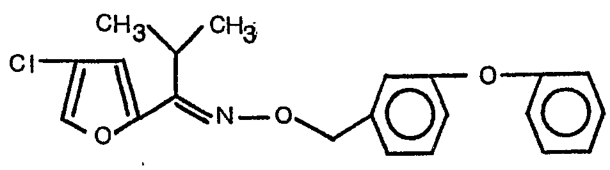

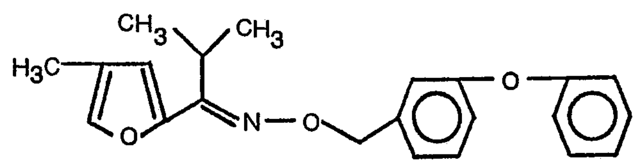

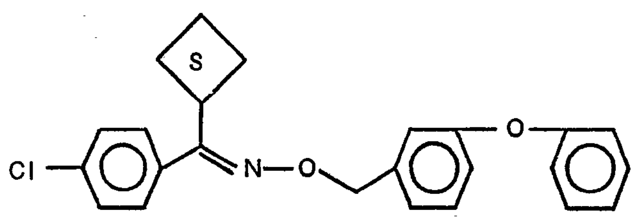

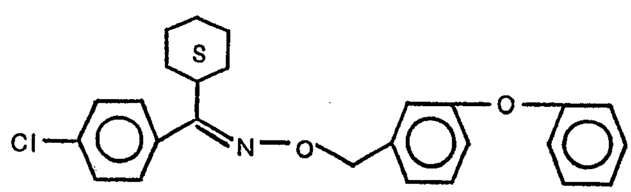

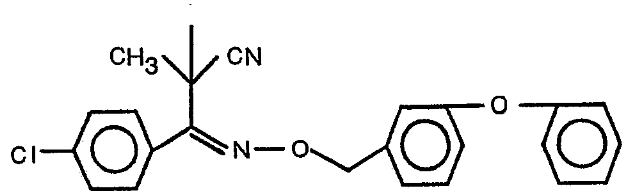

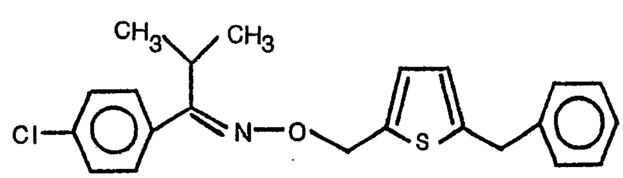

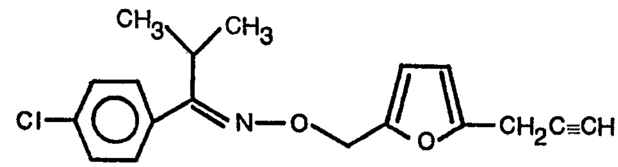

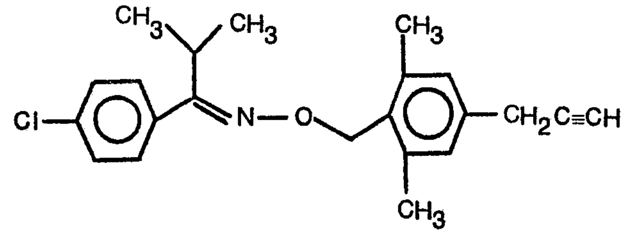

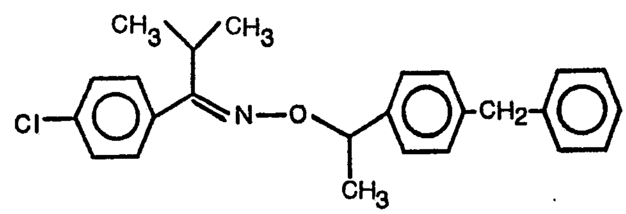

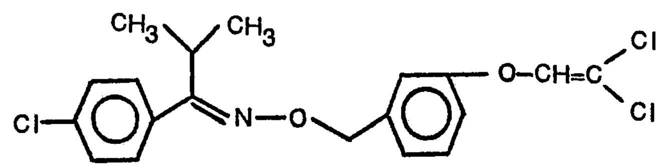

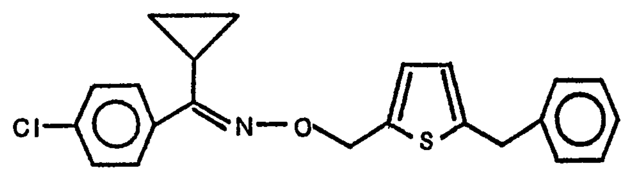

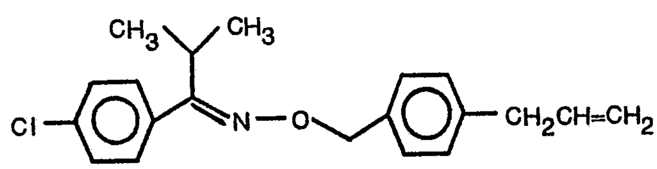

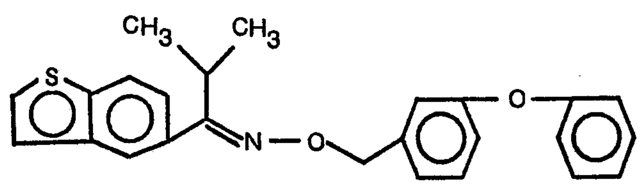

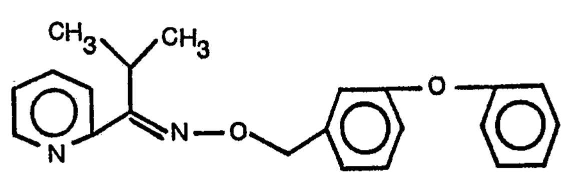

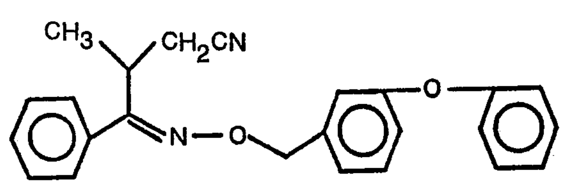

## SYNTHESIS METHODS

In general the compounds of this invention are prepared by two methods:

(a) Condensation of an aryl alkyl ketoxime with an alkyl halide in the presence of a base and a suitable solvent (general procedure E).
(b) Condensation of an isolated alkali metal ketoximate salt with an alkyl halide in an appropriate solvent (general procedure F).

The ketoximes are prepared from aryl alkyl ketones and hydroxylamine hydrochloride in a manner familiar to those skilled in the art and according to general procedure D.

The aryl alkyl ketones are obtained from commercial sources or are synthesized from available aromatics and carboxylic acid chlorides or from a benzonitrile and a Grignard reagent, as described in general procedures A, B and C.

The alkyl halides are obtained from commercial sources or are synthesized from available alcohol and a halogenating agent (e.g. thionyl chloride, phosphorous tribromide, etc.) in a manner familiar to those skilled in the art.

### *General Procedure A for Aryl Alkyl Ketones*

A solution of 3.0 mole of the substituted benzene and 0.8 mole of alkyl acid chloride is cooled to —5°C. Aluminum chloride (0.9 mole) is added portionwise, with vigorous stirring, while maintaining the reaction temperature at 0°C. The reaction mixture is brought to room temperature and allowed to stir overnight, whereupon the whole is poured into 450 ml of concentrated HCl and 1200 ml of ice-$H_2O$. The aqueous mixture is extracted two times with 350 ml portions of $CHCl_3$. The combined organic extracts are washed with 5% NaOH, $H_2O$, dried over $MgSO_4$ and concentrated under reduced pressure. The residue is distilled via short path to obtain 0.64—0.68 mole of pure product.

### *General Procedure B for Aryl Alkyl Ketones*

Typical Example

*Preparation of 5-bromo-2-isobutyrothiophenone*

A solution of 8.2 g (50 mole) of 2-bromothiophene and 5.3 g (50 mole) of isobutyryl chloride in 40 ml of $CS_2$ is cooled to 0°C. Aluminum chloride 7.3 g (55 mole) is added portionwise with vigorous stirring, while maintaining the reaction temperature at 0°C. The remaining manipulations are carried out as described in Procedure A to yield 6.2 g of pure product. Bp 70°C (0.02 mm); IR (neat) ($cm1^-$); 1666 (s), 1527 (s), 1412 (s), 1222 (s), 980 (s) nmr ($CDCl_3$) (ppm) 1.17 (6H, d), 3.33 (1H, m), 7.13—7.60 (2H, m).

### *General Procedure C for Aryl Alkyl Ketones*

Typical Example

*Preparation of m-Methyl isobutyrophenone*

A solution of 23.4 g (0.2 mole) of m-methylbenzonitrile in 50 ml of dry tetrahydrofuran is cooled to 10°C. A 2.2M solution (90 ml, 0.2 mole) of isopropyl magnesium chloride in ether is added dropwise, while maintaining the reaction temperature below 30°C. The ether is removed (by distillation) and the reaction mixture heated to 70°C for 4 hours. Upon cooling to room temperature, 100 ml of aqueous 6 N HCl is added dropwise. Reaction mixture is refluxed for 2 hours. The resulting two layers are separated and the aqueous layer is extracted three times with 200 ml portions of ether. The combined extracts are washed once with $H_2O$, dried over $MgSO_4$ and concentrated under reduced pressure. The residue is distilled using a vigreux column to obtain 23.6 g (73% yield) of pure product.

Procedure A was followed for the preparation of the aryl alkyl ketones listed in Table 1. The infrared (IR) and nuclear magnetic resonance (NMR) data are also given for each compound.

TABLE I

| Chemical Name | Synthesis Reactants | bp, °C/ pressure | ($\lambda$max) IR ($cm^{-1}$) | NMR (ppm) |
|---|---|---|---|---|
| p-chloroisobutryo-phenone | chlorobenzene, iso-butyryl chloride | 70°/0.02 mm. | 1690(s), 1587(s), 1389(d), 1220(s), 976(s), 840(s) | ($CDCl_3$) 1.15 (6H,d), 3.52 (1H,m) 7.38—8.05 (4H,m) |
| p-methylisobutryo-phenone | toluene, isobutyryl chloride | 85°/0.5 mm. | 1690(s), 1589(s), 1230(s), 980(s), 830(s) | ($CDCl_3$) 1.15 (6H,d) 2.35 (3H,s) 3.55 (1H,m), 7.23—802 (4H,m) |
| p-isopropylisobutryo-phenone | cumene, isobutyryl chloride | 80°/0.05 mm. | 1695(s), 1613(s), 1389(s), 1227(s), 980(s), 847(s) | ($CDCl_3$) 1.13 (6H,d), 1.26 (6H,d), 2.96 (1H,m), 3.57 (1H,m), 7.30—8.08 (4H,m) |
| p-ethoxyisobutryo-phenone | phenetole, isobutyryl chloride | 95°/0.3 mm. | 1695(s), 1615(s), 1234(s), 985(s), 847(s) | ($CDCl_3$) 1.10 (6H,d) 1.35 (3H,t), 3.57 (1H,m), 4.13 (2H,q) 6.93—8.10 (4H,m) |
| $\alpha$, p-Dichloropropio-phenone | chlorobenzene, 2-chloropropionyl chloride | 85°/0.03 mm. | 1690(s), 1587(s), 1087(s), 952(s), 840 | ($CDCl_3$) 1.7 (3H,d), 5.25 (1H,q), 7.45—8.13 (4H,m) |
| p-Fluoroisobutryo-phenone | fluorobenzene, isobutyryl chloride (molar ratio 3.2 to 1). | | 1680(s), 1597(s), 1389(d) 1214(s), 971(s), 844(s) | ($CDCl_3$) 1.2 (6H,d), 3.53 (1H,m) 7.03—8.22 (4H,m) |

*General Procedure D for Ketone Oximes*

In general, the aryl alkyl ketones prepared by general procedures A, B and C, were dissolved in ethanol and refluxed 3—5 hours in the presence of an excess of hydroxylamine hydrochloride and potassium hydroxide. The reaction mixture is concentrated and the oxime or its potassium salt is extracted into an organic solvent or $H_2O$, followed by acidification with concentrated HCl, respectively. Recrystallization from either hexane, hexane/$CHCl_2$ or $EtOH/H_2O$ followed.

This procedure was followed for the preparation of the aryl alkyl ketoximes listed in Table II. The physical properties (melting point, boiling point) and infrared (IR) and nuclear magnetic resonance (NMR) data are also known for each compound.

TABLE II

| Chemical Name | mp, °C | ($\lambda$max) IR ($cm^{-1}$) | NMR (ppm) |
|---|---|---|---|
| p-chloroisobutryo-phenone oxime[a] | 81—109° | 3279(br,s), 1610(s), 1495(s), 1089(s), 952(s), 830(d) | ($CDCl_3$) 1.13 (6H, Z isomer, d), 1.21 (6H, E isomer, D) 2.9 (1H, Z isomer, m), 3.7 (1H, E isomer, m), 7.4 (4H,s) 10.2 (1H,br,s) |
| p-methylisobutryo-phenone oxime[a] | 51—64° | 3279(br,s), 1626(s), 1522(s), 939(d), 820(s) | ($CDCl_3$) 1.11 (6H, Z isomer, d), 1.17 (6H, E isomer, d), 2.30 (3H,s) 2.87 (1H, Z isomer, m) 3.63 (1H, E isomer, m) 7.17—7.5 (4H,m), 10.0 (1H,br,s) |
| p-isopropylisobutryo-phenone oxime[a] | 74—106° | 3279(br,s), 1471(s), 1379(d), 948(s), 830(s) | ($CDCl_3$) 1.17 (12H, unsym t), 2.88 (1H,m), 2.87 (1H, Z isomer, m) 3.62 (1H, E isomer, m), 7.32 (4H,s), 10.15 and 10.27 (1H, br, both isomers) |
| p-chloroacetophenone oxime* | 97° | 3333(br,s), 1493(s), 1099(s), 930(s), 823(s) | ($CDCl_3$) 2.33 (3H,s), 7.38—7.78 (4H,m), 10.1 (1H,br,s) |
| p-chloropropiophenone oxime* | 65—66° | 3225(br,m), 1587(s), 1087(s), 966(s), 823(s) | ($CDCl_3$) 1.13 (3H,t), 2.85 (2H,q) 7.33—7.75 (4H,m), 10.33 (1H,br,s) |
| p-ethoxyisobutryo-phenone oxime[a] | 91—104° | 3279(br,s), 1613(s), 1515(s), 1250(br,s), 1047(br,s), 926(br,m), 837(br,s) | ($CDCl_3$) 1.08—1.55 (9H,m), 2.88 (1H, Z isomer, m), 3.65 (1H, E isomer, m), 4.11 (2H,q), 6.88—7.53 (4H,m), 9.27 (1H,br,s) |
| p-chlorobenzaldehyde oxime* | 98—104° | 3279(br,s), 1602(s), 1493(s), 1087(s), 971(s), 874(s) | ($CDCl_3$) 7.37—7.73 (4H,m), 8.23 (1H,s), 8.9 (1H,s) |

TABLE II (continued)

| Chemical Name | mp, °C | ($\lambda$max) IR ($cm^{-1}$) | NMR (ppm) |
|---|---|---|---|
| p-chlorophenyl cyclopropyl ketoxime* | 92—100° | 3279(br,m), 1613(s), 1475(s), 1093(s), 905(s), 827(s) | ($CDCl_3$) 0.57—1.23 (4H,m), 2.2 (1H,m), 7.37 (4H,s), 8.9 (1H,br,s) |
| isobutryophenone oxime*,[a] | 61—78° Bp 79—83°/ 0.05 mm | 3279(br,s), 1680(s), 943(s) 766, (br,s), 694(br,s) | ($CDCl_3$) 1.10 (6H, Z isomer, d) 1.21 (6H, E isomer, d), ].88 (1H, Z isomer, m), 3.68 (1H, E isomer, m), 7.32 (5H,s) 10.05 (1H,br,s) |
| $\alpha$-hydroxy-p-chloro-propiophenone oxime** | 113—115° | 3205(br,m), 1608(s), 1134(s) | ($Acd_6$) 1.43 (3H,d), 4.52 (1H,m) 5.55 (1H,m), 7.33—7.90 (4H,m) 10.33 (1H,br,s) |
| $\alpha$-methoxy-p-chloropro-piophenone oxime***,[a] | 111—112° | 3333(s), 1493(s), 1081(s), 922(s), 833(s) | ($Acd_6$) 1.19 (3H, one isomer, d) 1.41 (3H, one isomer, d), 3.20 (3H, one isomer, s), 3.38 (3H, one isomer, s), 4.18 (1H, one isomer, m), 5.25 (1H,q), 7.38—7.98 (4H,m) 10.35 (1H, one isomer, br, s), 10.87 (1H, one isomer, br, s) |
| p-fluoroisobutryo-phenone oxime[a] | 98—100° | 3225(br,s), 1613(s), 1220(s), 952(s), 844(s) | ($CDCl_3$) 1.11 (6H, Z isomer, d), 1.23 (6H, E isomer, d), 2.92 (1H, Z isomer, m), 3.67 (1H, E isomer, m), 7.07—7.60 (4H,m) |

[a] Two isomers (E and Z) obtained.

* Starting ketone obtained from commercial source.

** Prepared from $\alpha$, p-dichloropropiophenone and KOH.

*** Prepared from $\alpha$, p-dichloropropiophenone using $NH_2OH$—HCl and MeOH.

*General Procedure E for Oximinoethers*

In general, 0.02 mole of the ketone oximes prepared by general procedure D, were dissolved in 10 ml of toluene and added dropwise to a slurry of 0.02 mole of NaH (57% in mineral oil) in 10 ml toluene and 4 ml of DMF. The mixture is warmed to 50°C until $H_2$ evolution ceases, whereupon 0.02 mole of alkyl halide is added dropwise at 30°C. The reaction mixture is stirred overnight at room temperature and quenched by addition of 50 ml of $H_2O$ and 100 ml of toluene. The organic layer is washed with 5%, 50:50 aqueous/ethanol NaOH, followed by $H_2O$, dried over $MgSO_4$, and concentrated under reduced pressure to yield 0.015—0.017 mole of crude product. Chromatography on silica gel, using 50:50 $CH_2Cl_2$/hexane as eluant provides a highly purified product.

*General Procedure F for Oximinoethers*

A solution of 0.02 mole of ketoxime in 20 ml of ethanol is added to a freshly prepared ethanolic solution of 0.02 mole NaOEt. The mixture is stirred at room temperature for 30 minutes and concentrated under reduced pressure to dryness. The resulting sodium oximate salt is dissolved in a minimum volume of 90% DMF and 10% t-butanol, whereupon 0.02 mole of alkyl halide (neat) is added dropwise, causing an exotherm of 8°C. The reaction mixture is stirred overnight at room temperature and poured into $H_2O$. The resulting oil is extracted two times into toluene. The combined organic layers are washed with 5% NaOH (50:50 $H_2O$/ethanol) $H_2O$, dried over $MgSO_4$, and concentrated under reduced pressure to yield 0.15—0.19 mole of >90% pure product.

The oximino function of the compounds of this invention provides for the possibility of two geometrical isomers. This is shown in the following isomeric structures of a typical compound of this invention:

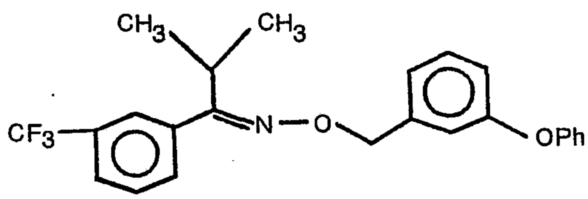

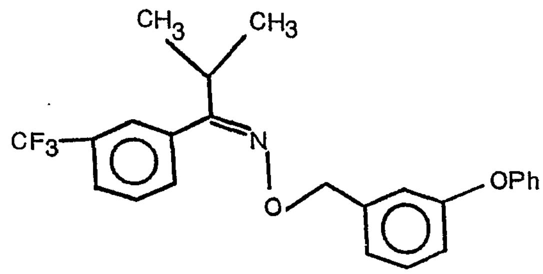

The O- (m-phenoxy) benzyl may be either cis to the isopropyl group (E or "syn" isomer) or cis to the m-trifluoromethyl phenyl ring (Z or "anti" isomer). Many compounds of this type occur as mixtures of isomers. In general, E isomer compounds or mixtures rich in E isomer compounds show the greater activity against some insect species and may be preferable. Z isomer compounds or mixtures rich in Z isomer compounds, however, do have substantial insecticidal activity. Accordingly, compounds having both isomeric structures of this invention are contemplated, regardless of isomer type or of isomer content in isomer mixtures.

Either of the above procedures were followed for the preparation of ketoximinoethers of this invention. Each compound, along with its method of synthesis (Procedure E or F), isomeric ratio, (E, Z), infrared (IR) and nuclear magnetic resonance (NMR) data are listed in Table III.

TABLE III

| Example Number | Chemical Name[a] (Synthesis Method) | Halide Reactant | E/Z ratio[b] | IR ($\lambda$max) $cm^{-1}$ | NMR (ppm) |
|---|---|---|---|---|---|
| 1 | 1-(4-chlorophenyl)-2-methyl-1-propanone, *O*-(5-(phenyl methyl)-3-furanyl) methyl Oxime (E) | 3-furylmethyl-5-benzyl-chloride | 65/35 | 1600(s), 1481(s) 1087(m), 826(s) | ($CDCl_3$) 1.08 (6H, Z isomer, d), 1.12 (6H, E isomer, d), 2.78 (1H, Z isomer, m), 3.48 (1H, E isomer, m), 3.92 (2H, s), 4.92 (2H, Z isomer, s), 5.02 (2H, E isomer, s), 6.03 (1H, Z isomer, s), 6.13 (1H, E isomer, s), 7.07—7.33 (10H,m) |
| 2 | 1-(4-chlorophenyl)-2-methyl-1-propanone, *O*-(5-(phenyl methyl)-3-furanyl) methyl Oxime (E) | 3-furylmethyl-5-benzyl-chloride | 65/35 | 1600(s), 1481(s) 1087(m), 826(s) | ($CDCl_3$) 1.06 (6H, Z isomer, d) 1.11 (6H, E isomer, d), 2.77 (1H, Z isomer, m), 3.48 (1H, E isomer, m), 3.90 (2H, s), 4.87 (2H, Z isomer, s), 5.02 (2H, E isomer, s), 6.03 (1H, Z isomer, s), 6.13 (1H, E isomer, s), 7.07—7.43 (10H, m) |
| 3 | 1-(4-chlorophenyl)-2-hydroxy-1-propanone, *O*-(3-phenoxyphenyl)-methyl Oxime (E) | m-phenoxy benzyl bromide | 100/0 | 3448(s), 1600(s) 1493(s), 1258(s) 8309(s) | ($CDCl_3$) 1.41 (3H,d), 1.61 (1H,br,s), 5.18 (1H,q), 5.25 (2H,s), 6.95—7.85 (13H,m) |

**TABLE III (continued)**

| Example Number | Chemical Name[a] (Synthesis Method) | Halide Reactant | E/Z ratio[b] | IR ($\lambda$max) $cm^{-1}$ | NMR (ppm) |
|---|---|---|---|---|---|
| 4 | 1-(4-chlorophenyl)-2-methyl-1-propanone, O-(1,3,4,5,6,7-Hexahydro-1,3-dioxo-2H-isoindol-2-yl) methyl Oxime (E) | N-hydroxymethyl, 3,4,5,6-tetrahydro phthalimide | 55/45 | 1739(s), 1613(s) 1504(s), 1361(s) 830(s) | ($CDCl_3$) 1.11 (6H, E and Z isomers, unsym. t) 1.80 (4H,br,s), 2.38 (4H,br,s), 2.81 (1H, Z isomer, m), 3.47 (1H, E isomer, m), 5.38 (2H, Z isomer, s), 5.50 (2H, E isomer, s), 7.07—7.63 (4H,m) |
| 5 | 1-(4-chlorophenyl)-2-methoxy-1-propanone, *O*-(3-phenoxyphenyl-methyl Oxime (F) | m-phenoxy-benzyl bromide | 84/16 | 1587(s), 1481(s) 1250(s), 830(s) | ($CDCl_3$) 1.17 (3H, S isomer, d), 1.38 (3H, E isomer, d), 3.10 (3H, E isomer, s), 3.33 (3H, Z isomer, s), 4.96—5.33 (2H, E and Z isomers, m), 6.83—7.93 (13H,m) |

[a] All oxime ethers are viscous liquids.
[b] Obtained from relative integration in the NMR of the methylene/methine protons derived from the halide reactant.

In order to enable those skilled in the art to better understand the nomenclature and for convenient reference, the structural formulae of the compounds named in Table III are set forth in the following Table IV.

TABLE IV

| Example Number | Structure |
|---|---|
| 1 | 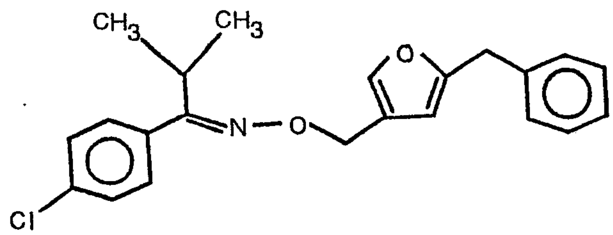 |
| 2 | Same as No. 1 — different isomer ratio |
| 3 | 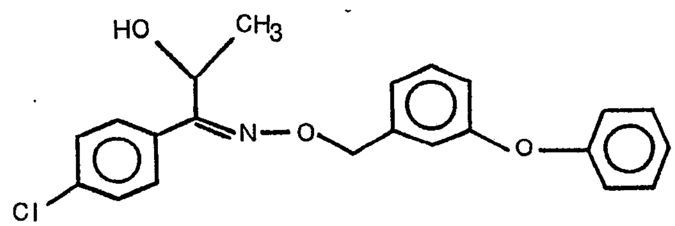 |
| 4 | 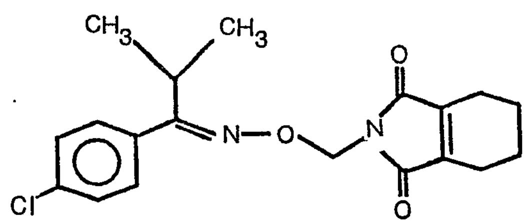 |
| 5 | 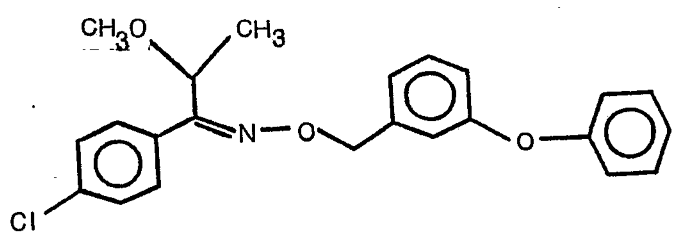 |

In an analogous manner, the following compound was prepared:

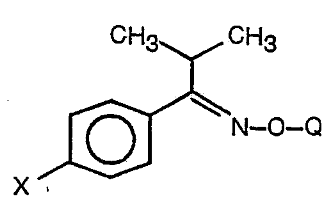

| Example Number | X | Q | E/Z ratio |
|---|---|---|---|
| 6 | Cl | 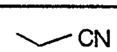 | 75/25 |

In an analogous manner, the following compound was prepared:

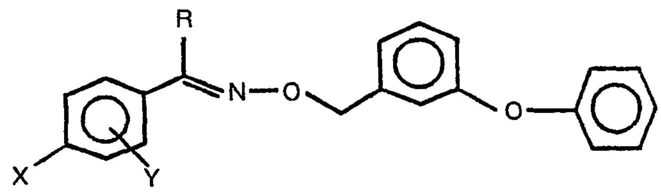

| Example Number | X | Y | R | E/Z ratio |
|---|---|---|---|---|
| 7 | H | m–$CF_3$ | iso-propyl | 55/45 |

In an analogous manner, the following compounds were prepared:

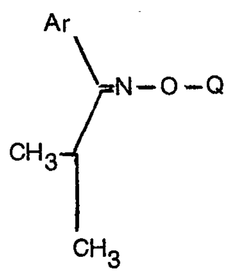

| Example Number | Ar | Q |
|---|---|---|
| 8 | Naphthyl | 3-phenoxybenzyl |
| 9 | indan-5-yl | 3-phenoxybenzyl |

The compounds of this invention have been found to exhibit considerable biological activity. They are especially potent pesticides when used to control or combat important agricultural pests. These compounds can be used in various ways to achieve biological action. They can be applied per se, as solids or in vaporized form, but are preferably applied as the toxic components in pesticidal compositions of the compound and a carrier. The compositions can be applied as dusts, as liquid sprays or as gas-propelled sprays and can contain, in addition to a carrier, additives such as emulsifying agents, wetting agents, binding agents, gases compressed to the liquid state, odorants, stabilizers and the like. A wide variety of liquid and solid carriers can be used in the pesticidal compositions. Non-limiting examples of liquid carriers include water; organic solvents such as alcohols, ketones, amides and esters; mineral oils such as kerosene, light oils and medium oils, and vegetable oils such as cottonseed oil. Non-limiting examples of solid carriers include talc, bentonite, diatomaceous earth, pyrophylite, fullers earth, gypsum, flours derived from cotton seeds and nut shells and various natural and synthetic clays having a pH not exceeding about 9.5.

The amount of the compounds of this invention utilized in pesticidal compositions will vary rather widely. It depends to some extent upon the type of composition in which the material is being used, the nature of the condition to be controlled and the method of application (i.e., spraying, dusting etc.). In the ultimate pesticidal composition, as applied in the field, pesticide concentrations as low as 0.0001 weight percent of the total composition can be used. In general, compositions, as applied, containing about 0.05 weight percent pesticide in either liquid or solid carrier give excellent results. In some cases, however, stronger dosages up to about 10 weight percent may be required.

In practice, pesticidal compositions are usually prepared in the form of concentrates, which are diluted in the field to the concentration desired for application. For example, the concentrate can be a wettable powder containing large amounts of the compound of this invention, a carrier (e.g., attapulgite or other clay), and wetting and dispersing agents. Such powders can be diluted prior to application, by dispersing it in water to obtain sprayable suspension containing the concentration of pesticide desired for application. Other concentrates can be solutions that can be later diluted, e.g., with kerosene. Thus, it is within the contemplation of this invention to provide pesticidal compositions containing up to

about 80 percent, by weight of the composition, of a pesticidal compound of this invention. Accordingly, depending upon whether it is ready for application or it is in concentrated form, the contemplated pesticidal compositions contain between about 0.0001 percent and about 80 percent, by weight of the compositions, of a pesticidal compound of this invention and a carrier, liquid or solid, as defined hereinbefore.

INSECTICIDE TEST METHODS
Bait Test [Housefly (adult)]

*Method of Treatment*

One milliliter of an aqueous solution of suspension of the candidate compound is pipetted into a 9 cm petri dish containing filter paper and 0.1 gm granular sugar. Ten adults are admitted and the dish is closed.

*Method of Recording Results*

Mortality is recorded after 24—75 hours. Compounds which product 90% mortality are reevaluated at lower concentrations in secondary tests. Mode of action may be by stomach poison, contact or vapor.

Larvicide/Growth Regulant Test
(Yellow Fever Mosquito larvae)

*Method of Treatment*

The rearing medium is treated prior to infestation. For mosquito larvae this consists of 10 ml of water containing 10 ppm of the candidate compound in a plastic cup. Food is added after infesting with 5 last instar larvae. The cup is then capped.

*Method of Recording Results*

Mortality (larvicide) is recorded after 24 hours.

Stomach Poison — Foliar Dip Test

*Primary Screen*

Southern Armyworm, (Larva)
Mexican Bean Beetle (Larva)

*Method of Treatment*

Lima bean leaves of a uniform size are momentarily dipped in a 500 ppm water-acetone of the test material. Treated leaves are placed on moistened filter paper in 9 cm petri dishes and allowed to air dry and then are infested. The dishes are then closed.

*Method of Recording Results*

Mortality is recorded 72 hours after infestation. Compounds active at 500 ppm are retested at 100 and 10 ppm.

All test results are recorded as percent mortality. In the tabulation of data, the insect species are abbreviated as follows: Housefly (HF), Mexican Bean Beetle (MB), Southern Armyworm (SA) and Yellow Fever Mosquito (YF).

The compounds of Examples 1 through 9 were subjected to the aforedescribed insecticide test. Test concentrations and results are set forth in Table V.

TABLE V

| COMPOUND | RATE (PPM) | HF | MB | SA | YF |
|---|---|---|---|---|---|
| Example 1 | 500 | 100 | 100 | 100 | — |
| | 100 | 90 | 100 | 85 | — |
| | 10 | 30 | 80 | 0 | 100 |
| | 1 | | — | — | 100 |
| Example 2 | 500 | 100 | 100 | 100 | — |
| | 100 | 100 | 100 | 95 | — |
| | 10 | 10 | 50 | 0 | 100 |
| | 1 | — | — | — | 100 |
| Example 3 | 500 | 30 | 80 | 70 | — |
| | 100 | — | 45 | 0 | — |
| | 10 | — | — | — | 100 |
| | 1 | — | — | — | 30 |
| Example 4 | 500 | 20 | 60 | 0 | — |
| | 100 | — | — | — | — |
| | 10 | — | — | — | 100 |
| | 1 | — | — | — | 0 |
| Example 5 | 500 | 90 | 100 | 100 | — |
| | 100 | 15 | 95 | 65 | — |
| | 10 | | | | |
| | 1 | | | | |
| Example 6 | 500 | 20 | 90 | 0 | |
| | 100 | | 0 | | |
| | 10 | | | | 100 |
| | 1 | | | | 10 |
| Example 7 | 500 | 100 | 100 | 90 | |
| | 100 | 20 | 95 | 15 | |
| | 10 | — | 80 | — | 100 |
| | 1 | | | | 70 |

TABLE V (continued)

| COMPOUND | RATE (PPM) | HF | MB | SA | YF |
|---|---|---|---|---|---|
| Example 8 | 500 | 20 | | 90 | |
| | 100 | — | | 15 | |
| | 10 | | | | |
| | 1 | | | | |
| Example 9 | 500 | 100 | 100 | 100 | |
| | 100 | 95 | 100 | 90 | |
| | 10 | | 75 | 35 | |
| | 1 | | | | |

**Claims**

1. Compounds having the formula:

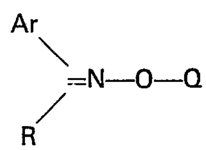

wherein Ar is phenyl; phenyl substituted in the 2- to 6-position with 1—5 halogen (Cl, Br, F, I), $C_1$—$C_4$ alkyl, amino, alkylamino, dialkylamino, alkoxy, alkylthio, methylenedioxy, ethylenedioxy, isopropylenedioxy, trifluoromethyl, haloalkyl, alkylthioalkyl, alkenyl, alkynyl, cyano, cyanoalkyl, carboalkoxy, alkylsulfonyl, alkoxyalkyl, haloalkenyl or acyl; indanyl, naphthyl, benzofuryl; benzodihydrofuryl, benzothienyl, heterocyclic aryl or heterocyclic aryl substituted with halogen, alkyl, nitro, amino, alkylamino, dialkylamino, alkoxy, methylenedioxy, alkylthio, haloalkyl, alkylthioalkyl, alkenyl, alkynyl, cyano, cyanoalkyl, carboalkoxy, alkylsulfonyl, alkoxyalkyl, haloalkenyl or acyl; R is $C_2$—$C_6$ alkyl, $C_3$—$C_6$ cycloalkyl or $C_2$—$C_6$ alkenyl any of which can be substituted by halogen, hydroxy, alkoxy, alkylthio, alkylsulfonyl, cyano, nitro, carboalkoxy or acyl; and Q is:

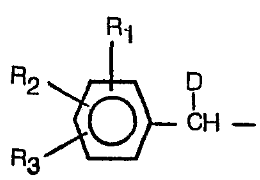

wherein D is hydrogen or alkyl; $R_1$ and $R_2$ are the same or different and are hydrogen, halogen, alkyl, alkenyl, alkynyl, methylenedioxy, haloalkyl, haloalkenyl or cyano; and $R_3$ is —ZY wherein Z is oxygen, sulfur or methylene and Y is hydrogen, alkyl, alkenyl, alkynyl or phenyl on which can be substituted alkyl, alkenyl, alkoxy, chlorine, bromine or fluorine; or

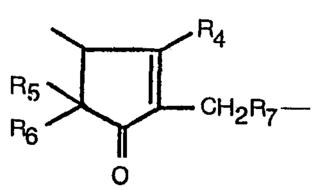

wherein $R_4$ is hydrogen or methyl; $R_5$ and $R_6$ are the same or different and are hydrogen or alkyl; and $R_7$ is ethenyl, ethynyl or butadienyl; or

$R_8CH_2$—

wherein $R_8$ is phthalimido, di- or tetra-hydrophthalimido or cyano or Q is

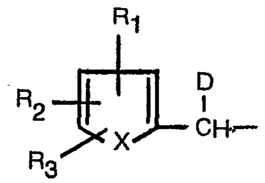

wherein X is oxygen or sulfur; $R_3$ is hydrogen or —ZY and $R_1$, $R_2$, D, Z and Y are defined as above provided that when Q is an $\alpha$-alkyl 3-phenoxybenzyl and R is alkyl or alkenyl of from two to six carbon atoms, optionally substituted by from one to a plurality of halogen atoms or is cyclopropyl, optionally substituted by one or two halogen atoms, Ar cannot be phenyl or phenyl substituted by one or two halogen atoms, alkyl of from one to four carbon atoms or alkoxy of from one to three carbon atoms, and provided also that, when Ar is an optionally substituted phenyl radical, and, at the same time Q is the 3-phenoxybenzyl radical and R is alkyl or alkenyl of from two to six carbon atoms optionally substituted by from one to a plurality of halogen atoms, or is cyclopropyl, optionally substituted by one or two halogen atoms, Ar is a phenyl radical substituted by a trifluoromethyl radical or a phenyl radical substituted by at least one alkoxy group and at least one alkyl group.

2. A compound of Claim 1 wherein said compound has the formula:

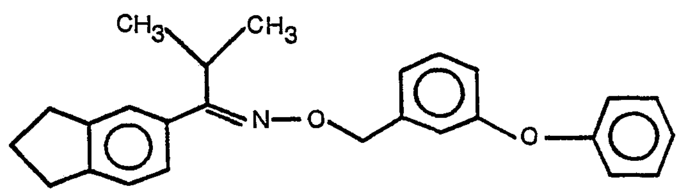

3. An insecticidal composition comprising a carrier and an insecticidally effective amount of a compound of either Claim 1 or Claim 2.

**Patentansprüche**

1. Verbindungen der Formel

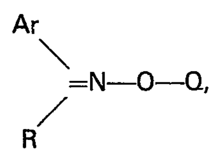

in der bedeuten:

Ar Phenyl; in 2-bis 6-Stellung mit 1 bis 5 Halogenatomen (Cl, Br, F, J), $C_1$- bis $C_4$-Alkyl, Amino, Alkylamino, Dialkylamino, Alkoxy, Alkylthio, Methylendioxy, Ethylendioxy, Isopropylendioxy, Trifluormethyl, Halogenalkyl, Alkylthioalkyl, Alkenyl, Alkinyl, Cyano, Cyanoalkyl, Carbalkoxy, Alkylsulfonyl, Alkoxyalkyl, Halogenalkenyl oder Acyl substituiertes Phenyl; Indanyl, Naphthyl, Benzofuryl, Benzodihydrofuryl, Benzothienyl, heterocyclisches Aryl oder mit Halogen, Alkyl, Nitro, Amino, Alkylamino, Dialkylamino, Alkoxy, Methylendioxy, Alkylthio, Halogenalkyl, Alkylthioalkyl, Alkenyl, Alkinyl, Cyano, Cyanoalkyl, Carbalkoxy, Alkylsulfonyl, Alkoxyalkyl, Halogenalkyl oder Acyl substituiertes heterocyclisches Aryl,

R $C_2$- bis $C_6$-Alkyl, $C_3$- bis $C_6$-Cycloalkyl oder $C_2$- bis $C_6$-Alkenyl, die jeweils mit Halogen, Hydroxy, Alkoxy, Alkylthio, Alkylsulfonyl, Cyano, Nitro, Carbalkoxy oder Acyl substituiert sein können, und

Q eine Gruppe

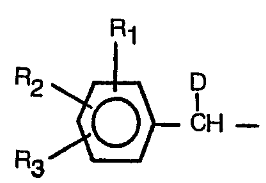

worin darstellen:

D H oder Alkyl,

$R_1$ und $R_2$ zugleich oder unabhängig H, Halogen, Alkyl, Alkenyl, Alkinyl, Methylendioxy, Halogenalkyl, Halogenalkenyl oder Cyano und

$R_3$ —ZY, wobei Z Sauerstoff, Schwefel oder Methylen und Y H, Alkyl, Alkenyl, Alkinyl oder Phenyl, das mit Alkyl, Alkenyl, Alkoxy, Chlor, Brom oder Fluor substituiert sein kann, oder eine Gruppe

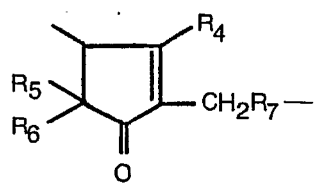

worin darstellen:

$R_4$ H oder Methyl,

$R_5$ und $R_6$ zugleich oder unabhängig H oder Alkyl, und

$R_7$ Ethenyl, Ethinyl oder Butadienyl,

oder eine Gruppe

$$R_8CH_2—,$$

worin $R_8$ Phthalimido, Dihydrophthalimido, Tetrahydrophthalimido oder Cyano darstellt, oder eine Gruppe

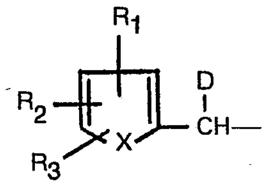

worin darstellen:

X Sauerstoff oder Schwefel,

$R_3$ H oder —ZY und

$R_1$, $R_2$, D, Z und Y dasselbe wie oben, mit der Maßgabe, daß, wenn Q ein $\alpha$-Alkyl-3-phenoxybenzyl ist und R Alkyl oder Alkenyl mit 2 bis 6 C-Atomen darstellt, die gegebenenfalls mit einem oder mehreren Halogenatomen substituiert sind, oder Cyclopropyl bedeutet, das gegebenenfalls mit einem oder zwei Halogenatomen substituiert ist, Ar nicht Phenyl oder mit einem oder zwei Halogenatomen, $C_1$- bis $C_4$-Alkyl oder $C_1$- bis $C_3$-Alkoxy substituiertes Phenyl bedeuten kann, sowie mit der Maßgabe, daß, wenn Ar gegebenenfalls substituiertes Phenyl darstellt und gleichzeitig Q 3-Phenoxybenzyl ist und R Alkyl oder Alkenyl mit 2 bis 6 C-Atomen, die gegebenenfalls mit einem oder mehreren Halogenatomen substituiert sind, oder Cyclopropyl darstellt, das gegebenenfalls mit einem oder zwei Halogenatomen substituiert ist, Ar ein mit einer Trifluormethylgruppe substituiertes oder mit mindestens einer Alkoxygruppe und mindestens einer Alkylgruppe substituiertes Phenyl darstellt.

2. Verbindung nach Anspruch 1, gekennzeichnet durch die Formel

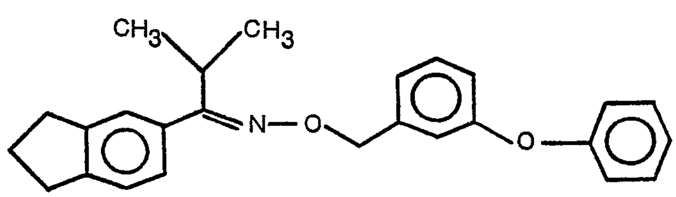

3. Insecticide Zusammensetzung, gekennzeichnet durch eine insecticid wirksame Menge einer Verbindung nach Anspruch 1 oder 2 sowie einen Träger.

**Revendications**

1. Composés caractérisés en ce qu'ils répondent à la formule:

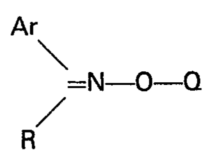

dans laquelle:

Ar est:

— phényle,

— phényle substitué sur les positions 2 à 6 par 1 à 5 halogènes (Cl, Br, F, I), alkyle ($C_1$—$C_4$),

amino, alkylamino, dialkylamino, alkoxy, alkylthio, méthylènedioxy, éthylenedioxy, isopropylendioxy, trifluorométhyle, halogènoalkyle, alkylthioalkyle, alcényle, alcynyle, cyano, cyanoalkyle, carboalkoxy, arylsulfonyle, alkoxyalkyle, halogénoalcényle ou acyle;

— indanyle; naphtyle; benzofuryle; benzodihydrofuryle; benzothiényle;

— aryle hétérocyclique;

— ou aryle hétérocyclique substitué par halogènes, alkyle, nitro, amino, alkylamino, dialkylamino, alkoxy, méthylènedioxy, alkylthio, halogènoalkyle, alkylthioalkyle, alcényle, alcynyle, cyano, cyanoalkyle, carboalkoxy, alkylsulfonyle, alkoxyalkyle, halogènoalcényle ou acyle;

R est alkyle ($C_2$—$C_6$), cycloalkyle ($C_3$—$C_6$) ou alcényle ($C_2$—$C_6$), chacun de ces radicaux pouvant être substitué par halogène, hydroxy, alkoxy, alkylthio, alkylsulfonyle, cyano, nitro, carboalkoxy ou acyle; et

Q représente le radical:

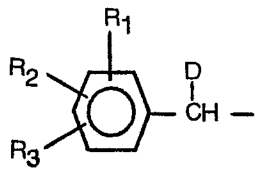

dans lequel D est hydrogène ou alkyle; $R_1$ et $R_2$, identiques ou différents, sont hydrogène, halogène, alkyle, alcènyle, alcynyle, méthylenedioxy, halogènoalkyle, halogènoalcényle ou cyano; et $R_3$ est le radical —ZY dans lequel Z est oxygène, soufre ou méthylène et Y est hydrogène, alkyle, alcényle, alcynyle ou phényle éventuellement substitué par alkyle, alcényle, alkoxy, chlore, brome ou fluor;

ou Q représente le radical:

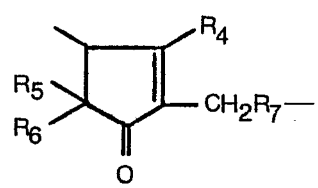

dans lequel $R_4$ est hydrogène ou méthyle, $R_5$ et $R_6$, identiques ou différents, sont hydrogène ou alkyle et R est éthényle, éthynyle ou butadiényle;

ou Q représente le radical:

$$R_8CH_2—$$

dans lequel $R_8$ est phtalimido, di ou tétra-hydrophtalimido; ou cyano;

ou Q représente le radical:

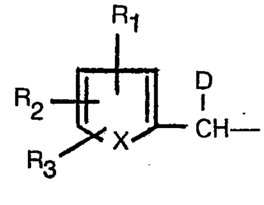

dans lequel X est oxygène ou soufre; $R_3$ est hydrogène ou le radical —ZY, étant entendu que $R_1$, $R_2$, D, Z et Y sont définis comme précédemment; sous réserve que, lorsque Q est un radical alpha-alkylphénoxy-3 benzyle et R, est alkyle ou alcényle contenant de 2 à 6 atomes de carbone, éventuellement substitué par un ou plusieurs atomes d'halogènes; ou un radical cyclopropyle éventuellement substitué par un ou deux atomes d'halogènes; Ar ne représente ni le radical phényle, ni un radical phényle substitué par de un à deux atomes d'halogènes ou radicaux alkyle contenant de 1 à 4 atomes de carbone ou alkoxy contenant de 1 à 3 atomes de carbone; et sous réserve aussi que, lorsque, Ar représente un radical phényle éventuellement substitué et que, Q et R, ont alors simultanément les valeurs suivantes:

— Q représente le radical phénoxy-3 benzyle,

— et R représente alkyle ou alcényle, comportant de 2 à 6 atomes de carbone, éventuellement substitué par un ou plusieurs atomes d'halogènes, ou cyclopropyle, éventuallement substitué par un ou deux atomes d'halogènes,

— Ar représente alors un radical phényle substitué par un radical trifluorométhyle, ou un radical phényle substitué par un moins un groupe alkoxy et au moins un groupe alkyle.

2. Composé selon la revendication 1, caractérisé en ce qu'il répond à la formule:

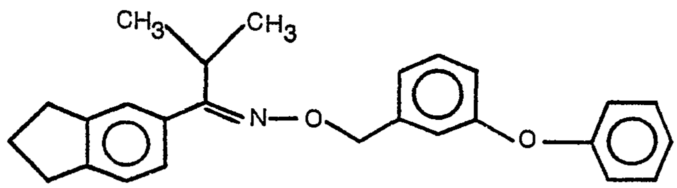

3. Composition insecticide comprenant un support et une quantité efficace vis-à-vis des insectes d'un composé selon l'une des revendications 1 ou 2.